# EUROPEAN PATENT APPLICATION

(11) **EP 4 403 640 A1**
(43) Date of publication of application: **24.07.2024**
(21) Application number: 22867262.2
(22) Date of filing: 31.08.2022
(51) Int. Cl.: C12Q 1/02, C12N 5/071, C12N 5/0783, C12N 5/09

(54) **METHOD FOR EVALUATING MIGRATORY ABILITY OF MIGRATORY CELLS**

(30) Priority: 13.09.2021 JP 2021148817
(71) Applicant: Toppan Holdings Inc., Tokyo 110-0016 (JP)
(72) Inventor: NADA, Isana, Tokyo 110-0016 (JP); MORIMURA, Rii, Tokyo 110-0016 (JP); KITANO, Shiro, Tokyo 110-0016 (JP)
(74) Representative: Plasseraud IP
(86) International application number: PCT/JP2022/032772
(87) International publication number: WO 2023/037940

(57) **Abstract**

The method for evaluating the migration ability of migratory cells includes: a culture step of culturing a cell structure having a stromal cell layer containing migratory cells and stromal cells, and a migratory cell-free cell layer containing no migratory cells on a top surface of the stromal cell layer; and an evaluation step of evaluating the migration ability of the migratory cells based on the mobility of the migratory cells in the cell structure after the culture step, the mobility of the migratory cells being a ratio of a height Hm of the migratory cells in the cell structure to a height Hs of the cell structure, the height Hm of the migratory cells in the cell structure being the distance between a point Pb and the migratory cells, the point Pb being an intersection where a perpendicular line down from the migratory cells to a bottom of the cell structure intersects the bottom of the cell structure, and the height Hs of the cell structure being the distance between the point Pb and a point Pt, the point Pt being an intersection where a straight line obtained by extending the perpendicular line to a top surface of the cell structure intersects the top surface of the cell structure.

## Description

### [Technical Field]

The present invention relates to a method for evaluating the migration ability of migratory cells, such as cytotoxic T cells, and a cell structure used in this method.

The present application claims priority to Japanese Patent Application No. 2021-148817 filed September 13, 2021, the entire contents of which are incorporated herein by reference.

### [Background Art]

The in vivo efficacy evaluation of medicines and their drug candidate compounds is performed using animals, such as mice and rats, before evaluation in humans. For example, when pharmaceutical companies and other organizations evaluate the efficacy of immune drugs, they generally use syngeneic mice (also referred to as allogeneic tumor-bearing mice). However, in addition to the species differences between mice and humans, it is difficult to evaluate a large number of drug candidate compounds in living mice because of inter-species differences and ethical issues. In order to solve these problems, cell structures that mimic the human organism by three-dimensionally organizing human-derived cells are being used for the evaluation of drug efficacy.

On the other hand, cytotoxic T cells (i.e., CTLs) among immune cells infiltrate into the interstitial tissue around cancer, from the blood, and migrate to the tumor site (tumor-infiltrating lymphocytes, also referred to as TILs). In general, it is known that cancer immunotherapy is more likely to be successful when CTLs are concentrated in the vicinity of the cancer (e.g., NPL 1 and NPL 2). Therefore, the behavior of TILs is of interest as a target to determine whether immune checkpoint inhibitors are effective. However, despite the importance of TILs in human body, molecular-targeted drugs used for cancer immunotherapy etc. and immune cell drugs for CAR-T (chimeric antigen receptor-T cell) therapy etc. have not been evaluated for TILs at the drug discovery stage. It is the mainstream that these drug candidate compounds are selected based solely on their affinity to the target cancer cells and then evaluated in syngeneic mice.

As a method for evaluating the migration ability of migratory cells ex vivo, for example, there is an in vitro model to evaluate the infiltration of CTLs into a spheroid-like cell structure of cancer cells by culturing the cell structure in a culture medium containing CTLs (NPL 3).

### [Citation List]

### [Non-Patent Literature]

NPL 1: Santoiemma and Powell, Cancer Biology & Therapy, 2015, vol. 16 (6), pp. 807-820.
NPL 2: Lin et al., Biomedicine & Pharmacotherapy, 2020, vol. 132, 110873
NPL 3: Herter et al., Cancer Immunology Immunotherapy, 2017, vol. 66, pp. 129-140.
NPL 4: Romo et al., Immunology, 2016, vol. 148, pp. 125-139.
NPL 5: Rouse and Sehrawat, NATURE REVIEWS, 2010, vol. 10, pp. 515-526.
NPL 6: Hughes and Nibbs, FEBS Journal, 2018, vol. 285, pp. 2944-2971.
NPL 7: Kochin, et al., Oncoimmunology, 2017, vol. 6 (4), e1293214.

### [Summary of the Invention]

### [Technical Problem]

Since the cell structure disclosed in NPL 3 is a three-dimensional tissue that does not contain stromal cells, it is not possible to evaluate how immune cells infiltrate into the interstitial tissue and reach cancer cells, as is the case in vivo.

If TILs can be evaluated more appropriately at the drug discovery stage, it will contribute to the efficiency of drug development. For this reason, it is desirable to develop a cell structure more suitable for evaluating TILs. That is, the problem to be solved by the present invention is, for example, to provide a method for evaluating the migration ability of migratory cells, such as cytotoxic T cells, and a cell structure used in this method.

### [Solution to Problem]

As a result of conducting intensive research to solve the above problem, the present inventors found that a cell structure produced by laminating, on the top surface of a stromal cell layer containing migratory cells and stromal cells, a migratory cell-free cell layer containing no migratory cells can be cultured to be able to evaluate the migration ability of the migratory cells based on the position of the migratory cells in the cultured cell structure. Thus, the present invention has been completed.

A method for evaluating the migration ability of migratory cells according to a first aspect of the present invention includes: a culture step of culturing a cell structure having a stromal cell layer containing migratory cells and stromal cells, and a migratory cell-free cell layer containing no migratory cells on a top surface of the stromal cell layer; and an evaluation step of evaluating the migration ability of the migratory cells based on the mobility of the migratory cells in the cell structure after the culture step, the mobility of the migratory cells being a ratio of a height Hm of the migratory cells in the cell structure to a height Hs of the cell structure, the height Hm of the migratory cells in the cell structure being the distance between a point Pb and the migratory cells, the point Pb being an intersection where a perpendicular line down from the migratory cells to a bottom of the cell structure intersects the bottom, and the height Hs of the cell structure being the distance between the point Pb and a point Pt, the point Pt being an intersection where a straight line obtained by extending the perpendicular line to a top surface of the cell structure intersects the top surface of the cell structure.

A cell structure according to a second aspect of the present invention is a cell structure including a stromal cell layer containing migratory cells and stromal cells and a migratory cell-free cell layer containing no migratory cells on a top surface of the stromal cell layer, the migratory cell-free cell layer containing migration target cells, and the migration target cells being cells that determine the migration direction of the migratory cells.

A cell structure according to a third aspect of the present invention is a cell structure including a stromal cell layer containing migratory cells and stromal cells and a migratory cell-free cell layer containing no migratory cells on a top surface of the stromal cell layer, the migratory cell-free cell layer containing no migration target cell.

### [Advantageous Effects of the Invention]

In the cell structure according to the present embodiment, migratory cells, such as CTLs, are contained inside the cell structure composed of stromal cells, and the migratory cells migrate within the cell layer of the stromal cells. Therefore, the method for evaluating the migration ability of migratory cells according to the present embodiment can more accurately evaluate the migration ability of migratory cells inside the interstitial tissue by using this cell structure.

### [Brief Description of the Drawing]

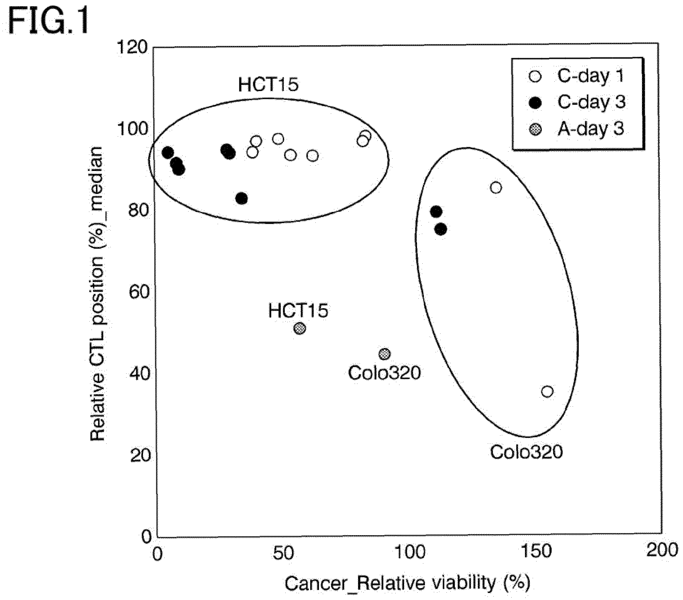

Fig. 1 is a diagram showing the relationship between the CTL mobility (%) of cell structures C and A, and the relative viability (%) of cancer cells in Example 1.

### [Description of the Embodiments]

In the present embodiment and the present specification, the "cell structure" is a three-dimensional structure in which multiple cell layers are laminated. The "cell layers" refer to layers, in a cross-sectional image of a section of the cell structure, which is observed at a magnification at which a cell nucleus can be recognized, that is, at which the entire thickness of the nuclear-stained section is included in the field of view, composed of a group of cells and stroma that are present in a direction perpendicular to the thickness direction with the cell nuclei not overlapping each other in the thickness direction. In addition, "layered" means that two or more different cell layers are stacked in the thickness direction.

### <Cell structure>

The cell structure according to one embodiment of the present invention is a three-dimensional structure having a stromal cell layer containing migratory cells and stromal cells, and a migratory cell-free cell layer containing no migratory cells on the top surface of the stromal cell layer. In the living body, cancer tissue is formed in contact with interstitial tissue, and migratory immune cells, such as CTLs, migrate within the interstitial tissue to reach the cancer tissue. Therefore, in order to evaluate migration ability in vivo, it is necessary to evaluate the mode of migration of migratory cells in a three-dimensional tissue that mimics the interstitial tissue as in the living body. The cell structure according to the present embodiment is a cell structure that contains migratory cells within a stromal cell layer and mimics the interstitial tissue and cancer tissue in vivo. Therefore, the cell structure is suitable as a cell model for evaluating the migration ability of migratory cells in vivo.

The cell structure according to one embodiment of the present invention is preferably a three-dimensional structure having a stromal cell layer containing migratory cells and stromal cells, and a migratory cell-free cell layer containing no migratory cells on the top surface of the stromal cell layer, wherein the migratory cell-free cell layer is located on the top surface of the cell structure.

The migratory cells used in the present embodiment are not particularly limited, as long as they are cells having migration ability. Examples of the migratory cells include immune cells such as lymphocyte cells, metastatic cancer cells, and the like. Examples of lymphocyte cells include T cells, B cells, NK cells, macrophages, dendritic cells, and the like. The migratory cells used in the present invention are particularly preferably CTLs that play an important role in the efficacy of cancer immunotherapy drugs and immune cell drugs. The type of migratory cells contained in the stromal cell layer of the cell structure according to the present embodiment may be one, or may be two or more.

The migratory cell-free cell layer in the cell structure according to the present embodiment is contained in the stromal cell layer, and is not particularly limited as long as it is a cell layer that does not contain the target migratory cells whose migration ability is to be evaluated. The migratory cell-free cell layer may be a cell layer containing migration target cells (a migration target cell layer) or a cell layer containing no migration target cell (a migration target cell-free layer). In the following, among the cell structures according to the present embodiments, the cell structure in which the migratory cell-free cell layer is a migration target cell layer is also referred to as "the first cell structure," and the cell structure in which the migratory cell-free cell layer is a migration target cell-free layer is also referred to as "the second cell structure." The phrase "the cell structure according to the present embodiment" without specific mention includes both the first cell structure and the second cell structure.

In the present embodiment and the present specification, the migration target cells refer to cells that determine the migration direction of migratory cells. Specifically, the migration target cells refer to cells that are attractive to migratory cells, and cells that are repellent to migratory cells. Examples of the migration target cells include cancer cells (NPL 1 and NPL 2), infected cells infected with viruses, bacteria, etc. (NPL 4 and NPL 5), and cells that produce chemokines due to inflammation or the like (NPL 6). Further, the cancer cells refer to cells that have been derived from somatic cells and acquired infinite proliferation potential. The type of migration target cells contained in the cell structure of the present embodiment may be one, or may be two or more.

In the cell structure according to the present embodiment, when the migration target cells are cancer cells, the cancer cells may be established culture cells, or cancer cells obtained from a cancer patient. The cancer cells obtained from a cancer patient may be previously grown by culture. Specifically, examples of the cancer cells include primary cancer cells obtained from a cancer patient, artificially cultured cancer cells, iPS cancer stem cells, cancer stem cells, and established cancer cells previously prepared for use in studies of cancer therapy and development of anticancer drugs. Moreover, the cancer cells may be derived from a human, or may be derived from an animal other than a human. When the cell structure according to the present embodiment contains cancer cells obtained from a cancer patient, cells other than the cancer cells obtained from the cancer patient may be contained together with the cancer cells. Examples of the cells other than cancer cells include one or more types of cells contained in solid tissue removed after surgery.

Non-limiting examples of the cancer from which cancer cells are derived to be included in the cell structure according to the present embodiment include breast cancer (e.g., invasive ductal carcinoma, ductal carcinoma in situ, and inflammatory breast cancer), prostate cancer (e.g., hormone-dependent prostate cancer and hormone-independent prostate cancer), pancreatic cancer (e.g., pancreatic duct cancer), gastric cancer (e.g., papillary adenocarcinoma, mucinous adenocarcinoma, and adenosquamous carcinoma), lung cancer (e.g., non-small-cell lung cancer, small-cell lung cancer, and malignant mesothelioma), colon cancer (e.g., gastrointestinal stromal tumor), rectal cancer (e.g., gastrointestinal stromal tumor), colorectal cancer (e.g., familial colorectal cancer, hereditary nonpolyposis colorectal cancer, and gastrointestinal stromal tumor), small intestinal cancer (e.g., non-Hodgkin's lymphoma and gastrointestinal stromal tumor), esophageal cancer, duodenal cancer, tongue cancer, pharyngeal cancer (e.g., nasopharyngeal cancer, oropharyngeal cancer, and hypopharyngeal cancer), head and neck cancer, salivary gland cancer, brain tumor (e.g., pineal astrocytoma, pilocytic astrocytoma, diffuse astrocytoma, and anaplastic astrocytoma), neurilemmoma, liver cancer (e.g., primary liver cancer and extrahepatic bile duct cancer), renal cancer (e.g., renal cell cancer and transitional cell cancer of the renal pelvis and ureter), gallbladder cancer, bile duct cancer, pancreatic cancer, hepatoma, endometrial cancer, cervical cancer, ovarian cancer (e.g., epithelial ovarian cancer, extragonadal germ cell tumor, ovarian germ cell tumor, and ovarian low-malignant potential tumor), bladder cancer, urethral cancer, skin cancer (e.g., intraocular (ocular) melanoma and Merkel cell carcinoma), hemangioma, malignant lymphoma (e.g., reticulosarcoma, lymphosarcoma, and Hodgkin's disease), melanoma (malignant melanoma), thyroid cancer (e.g., medullary thyroid cancer), parathyroid cancer, nasal cancer, paranasal cancer, bone tumor (e.g., osteosarcoma, Ewing's tumor, uterine sarcoma, and soft-tissue sarcoma), metastatic medulloblastoma, hemangiofibroma, dermatofibrosarcoma protuberans, retinal sarcoma, penile cancer, testicular tumor, pediatric solid cancer (e.g., Wilms tumor and pediatric renal tumor), Kaposi sarcoma, Kaposi sarcoma caused by AIDS, tumor of the maxillary sinus, fibrous histiocytoma, leiomyosarcoma, rhabdomyosarcoma, chronic myeloproliferative disorders, leukemia (e.g., acute myelogenous leukemia and acute lymphoblastic leukemia), and the like.

When the migration target cells contained in the first cell structure are attractive to migratory cells, the migratory cells in the cell structure migrate toward the migration target cell layer. When the migration target cells are repellent to migratory cells, the migratory cells in the cell structure migrate in the direction away from the migration target cell layer, that is, toward the bottom of the stromal cell layer. On the other hand, in the second cell structure, the migratory cells in the cell structure randomly migrate within the cell structure; thus, after the culture step, the migratory cells are present randomly throughout the entirety of the cell structure.

In the present embodiment and the present specification, the "stromal cells" refer to "cells that constitute the stroma and are cells other than the target migratory cells whose migration ability is to be evaluated." Examples of the stromal cells contained in the cell structure according to the present embodiment include fibroblasts, endothelial cells, pericytes, immune cells, inflammatory cells, and the like. The type of stromal cells contained in the cell structure according to the present embodiment may be one, or may be two or more. The type of stromal cells contained in the cell structure according to the present embodiment is not particularly limited, and can be suitably selected in consideration of the type of migratory cells to be contained, the type of migration target cells to be used for evaluation, the in-vivo environment in which desired migration occurs, and the like.

The stromal cells that constitute the stromal cell layer of the cell structure according to the present embodiment are preferably one or more types selected from the group consisting of fibroblasts, immune cells other than the migratory cells mentioned above, and mast cells. In particular, it is preferable that at least fibroblasts are contained, because they can more closely resemble the tissue environment in vivo. The stromal cell layer of the cell structure according to the present embodiment may be a cell layer consisting of fibroblasts and migratory cells, a cell layer consisting of two or more types of stromal cells, including fibroblasts, and migratory cells, or a cell layer consisting of one or two or more types of stromal cells, including fibroblasts, migratory cells, and other cells.

The blood vessel network structure and lymphatic network structure are important for simulating the migration of migratory cells and the in-vivo environment. Therefore, the cell structure according to the present embodiment may include a vascular network structure. Namely, the cell structure according to the present embodiment is preferably one in which a vascular network structure such as of lymphatic vessels and/or blood vessels is three-dimensionally formed inside the stromal cell layer to form tissues closer in structure to those in vivo. In the present embodiment and the present specification, the term "vascular network structure" refers to a network structure, such as a blood vessel network or a lymphatic vessel network, in living tissues.

A vascular network structure can be formed by incorporating endothelial cells, which constitute vessels, as stromal cells. The endothelial cells contained in the stromal cell layer of the cell structure according to the present embodiment may be vascular endothelial cells or lymphatic endothelial cells. Moreover, both vascular endothelial cells and lymphatic endothelial cells may be contained.

The number of endothelial cells in the stromal cell layer of the cell structure according to the present embodiment is not particularly limited as long as it is sufficient for forming a vascular network structure, and can be determined as appropriate in consideration of the size of the cell structure, the types of endothelial cells and cells other than endothelial cells, and the like. For example, a cell structure having a stromal cell layer in which a vascular network structure is formed can be prepared by setting the abundance ratio (cell number ratio) of endothelial cells to all the cells constituting the stromal cell layer of the cell structure according to the present embodiment to 0.1% or more. When fibroblasts are used as the cells other than endothelial cells, the number of endothelial cells in the cell structure according to the present embodiment is preferably 0.1% or more, and more preferably 0.1% to 5.0%, of the number of fibroblasts. When both vascular endothelial cells and lymphatic endothelial cells are contained as endothelial cells, the total number of vascular endothelial cells and lymphatic endothelial cells is preferably 0.1% or more, and more preferably 0.1% to 5.0%, of the number of fibroblasts.

The stromal cell layer of the cell structure according to the present embodiment may also contain one or more cells other than the migratory cells and stromal cells. The migration target cell layer of the cell structure according to the present embodiment may also contain one or more cells other than the migration target cells. Such other cells are not particularly limited, as long as they do not affect the migration of migratory cells in the stromal cell layer. Examples of such other cells include nerve cells, mast cells, epithelial cells, cardiac muscle cells, smooth muscle cells, bone cells, liver cells, spleen cells, pancreatic islet cells, pancreatic cells, alveolar epithelial cells, tissue stem cells, and the like. Such other cells may also be cancer cells that are not attractive or repellent to migratory cells whose migration ability is to be evaluated, inflammatory cells, or migratory cells other than the migratory cells whose migration ability is to be evaluated. The migration target cell-free layer of the second cell structure according to the present embodiment is composed of one or more of the other cells.

The cells including stromal cells, migratory cells, and migration target cells that constitute the cell structure according to the present embodiment are not particularly limited. The cells may be cells obtained from animals, cells obtained by culturing cells obtained from animals, cells obtained by applying various treatments to cells obtained from animals, or cultured cell lines. In the case of cells obtained from animals, the sampling site is not particularly limited. The cells may be somatic cells derived from bone, muscle, viscus, nerve, brain, bone, skin, blood, etc., reproductive cells, or embryonic stem cells (ES cells). Further, the organism species from which the cells constituting the cell structure according to the present embodiment are derived is not particularly limited. The cells can be derived from humans or animals such as monkeys, dogs, cats, rabbits, pigs, cows, mice, and rats. The cells obtained by culturing cells obtained from animals may be primary cultured cells or subcultured cells. Further, the cells obtained by applying various treatments may include induced pluripotent stem cells (iPS cells) or cells after differentiation induction. The cell structure according to the present embodiment may be composed of only cells derived from the same organism species, or cells derived from several types of organism species.

The migratory cells in the cell structure according to the present embodiment may be produced to be evenly distributed throughout the stromal cell layer, or may be produced to be present in a specific cell layer of the stromal cell layer. Since the variation in the mobility of each migratory cell in the cell structure is reduced, the cell structure according to the present embodiment is preferably one in which migratory cells are present in a specific cell layer of the stromal cell layer. In particular, in terms of easily reflecting the level of migration ability on mobility, a cell structure is preferred in which migratory cells are present within the range from a middle part to the bottom of the stromal cell layer, and a cell structure in which migratory cells are present near the bottom of the stromal cell layer is more preferred.

The cell structure according to the present embodiment can be produced by first forming a stromal cell layer on a substrate, such as a cell container, and then forming a migratory cell-free cell layer on the top surface of the stromal cell layer. The methods for producing the stromal cell layer and the migratory cell-free cell layer are not particularly limited. For example, the method may be a production method including sequentially laminating each layer, a method including forming two or more cell layers at once, or a method including forming multiple cell layers by suitably combining both production methods. For example, the method used may be a production method in which a layer is formed for every type of cell, and the resulting cell layers are sequentially laminated, or a method in which a cell mixed solution containing a mixture of several types of cells is previously prepared, and a multilayer cell structure is produced at once from the cell mixed solution.

An example of the method of producing the cell structure by laminating each layer in sequence includes the method disclosed in JP 4919464 B, that is, repeating alternately a step of forming a cell layer and a step of bringing the formed cell layer into contact with a solution containing an extracellular matrix (ECM) component to laminate the cell layers in a continuous manner. For example, when performing this method, a cell mixture in which all the cells forming a cell structure are mixed is prepared beforehand, and then individual cell layers are formed using this cell mixture, thereby producing a cell structure. Further, the cell structure can also be produced by forming each cell layer for every type of cell.

As the method for forming two or more cell layers at once, for example, the method disclosed in JP 5850419 B can be used. Specifically, this method produces a cell structure having multiple cell layers by previously coating the entire cell surface with a polymer containing an arginine-glycine-aspartic acid (RGD) sequence bound to integrin, and a polymer interacting with the polymer containing the RGD sequence, accommodating the cells coated with an adhesive film in a cell culture container, and then integrating the coated cells by centrifugation or the like. For example, when performing this method, a cell mixture is prepared beforehand by mixing all the cells forming a cell structure, and then coated cells are prepared by adding an adhesive component to the cell mixture, followed by one centrifugation, thereby producing a cell structure. Furthermore, for example, cells coated with endothelial cells and cells coated with fibroblasts are separately prepared. Then, a multilayer formed of the cells coated with fibroblasts is formed. Then, a layer formed of the cells coated with endothelial cells is laminated on the multilayer formed of the cells coated with fibroblasts, followed by further laminating thereon another multilayer formed of the cells coated with fibroblasts, thereby forming a thick cell structure including a vascular network structure sandwiched between the fibroblast layers.

The stromal cell layer and migratory cell-free cell layer of the cell structure according to the present embodiment can be easily produced by a method in which a cell mixture obtained by suspending cells in a solution containing at least a cationic buffer solution, an extracellular matrix component, and a polyelectrolyte is collected on a substrate, followed by culturing (WO2017/183673, JP 6427836 B).

Specifically, for example, a method having the following steps (A) to (C) can be used for the production. After steps (A) and (B) are performed at least once, step (C) is performed.
Step (A) of obtaining a mixture in which cells are suspended in a solution containing at least a cationic substance, an extracellular matrix component, and a polyelectrolyte;
step (B) of collecting the cells from the obtained mixture and forming a cell aggregate on a substrate; and
step (C) of culturing the cell aggregate to obtain a cell structure.

In the present embodiment and the present specification, the term "cell aggregate" refers to a population of cells. The cell aggregates also include cell precipitates obtained by centrifugation, filtration, or the like (aggregates of cells formed by precipitating cells). In an embodiment, the cell aggregates are in the form of a slurry-like viscous body. In the present specification, the "slurry-like viscous body" refers to gel-like cell aggregates, as described in Akihiro Nishiguchi et al., Macromol Biosci. 2015 Mar; 15 (3): 312-7.

Step (A) is performed by suspending cells that are used to produce the cell structure in a solution containing at least a cationic substance, an extracellular matrix component, and a polyelectrolyte (hereinafter also referred to as "the solution for cell suspension"). The cell population suspended in the cell suspension solution may be all or part of the cells that are used to produce the cell structure.

The solution for cell suspension is prepared by dissolving a cationic substance, an extracellular matrix component, and a polyelectrolyte in a solvent. The solvent for preparing a cell suspension is not particularly limited, as long as it does not have toxicity to the cells, and does not impair proliferative properties or function. Water, buffer solutions, cell culture media, etc., can be used. Examples of the buffer solution include phosphate buffered saline (PBS), HEPES, Hanks buffer solution, and the like. Examples of the culture medium include D-MEM, E-MEM, MEMα, RPMI-1640, Ham's F-12, and the like.

As the cationic substance, any positively charged substance can be used, as long as it does not adversely affect the cell growth and the formation of cell aggregates. Examples of cationic substances include cationic buffer solutions, such as tris-hydrochloric acid buffer solution, tris-maleic acid buffer solution, bis-tris buffer solution, and HEPES; ethanolamine, diethanolamine, triethanolamine, polyvinylamine, polyallylamine, polylysine, polyhistidine, polyarginine, and the like. The cationic substance used in the production of the cell structure according to the present embodiment is preferably a cationic buffer solution.

The concentration of the cationic substance is not particularly limited, as long as it does not adversely affect the cell growth and the formation of cell aggregates. The concentration of the cationic substance in the solution for cell suspension used in step (A) is preferably 10 to 100 mM, more preferably 20 to 90 mM, even more preferably 30 to 80 mM, still even more preferably 40 to 70 mM, still even more preferably 45 to 60 mM, and still even more preferably 50 mM.

When a cationic buffer solution is used as the cationic substance, the pH of the cationic buffer solution is not particularly limited, as long as it does not adversely affect the cell growth and the formation of cell aggregates. The pH of the cationic buffer solution used in the solution for cell suspension is preferably 6.0 to 8.0, more preferably 7.0 to 8.0, even more preferably 7.2 to 7.6, and particularly preferably 7.4.

In the present embodiment and the present specification, the term "polyelectrolyte" refers to a polymer with a dissociable functional group in the polymer chain. As the polyelectrolyte used in the present embodiment, any polyelectrolyte can be used, as long as it does not adversely affect the cell growth and the formation of the cell structure. Examples of polyelectrolytes include, but are not limited to, glycosaminoglycans such as heparin, chondroitin sulfate (e.g., chondroitin 4-sulfate and chondroitin 6-sulfate), heparan sulfate, dermatan sulfate, keratan sulfate, and hyaluronic acid; dextran sulfate, rhamnan sulfate, fucoidan, carrageenan, polystyrene sulfonic acid, polyacrylamido-2-methylpropanesulfonic acid, polyacrylic acid, and the like. The mixture prepared in step (A) may be mixed with only one polyelectrolyte, or two or more polyelectrolytes in combination. In the production of the cell structure according to the present embodiment, it is preferable to use glycosaminoglycans, and it is more preferable to use heparin and/or dextran sulfate. The amount of the polyelectrolyte mixed in the solution for cell suspension is not particularly limited, as long as it does not adversely affect the cell growth and the production of the cell structure. For example, the concentration of the polyelectrolyte in the solution for cell suspension may be more than 0 mg/mL, preferably 0.010 mg/mL or more, more preferably 0.020 mg/mL or more, even more preferably 0.025 mg/mL or more, and still even more preferably 0.05 mg/mL or more. Further, the concentration of the polyelectrolyte in the solution for cell suspension is preferably less than 1.0 mg/mL, more preferably 0.75 mg/mL or less, even more preferably 0.5 mg/mL or less, still even more preferably 0.25 mg/mL or less, and particularly preferably 0.1 mg/mL or less.

As the extracellular matrix component, any component that constitutes an extracellular matrix (also referred to as ECM) can be used, as long as it does not adversely affect the cell growth and the formation of cell aggregates. Examples include collagen, laminin, fibronectin, vitronectin, elastin, tenascin, entactin, fibrillin, proteoglycan, glycosaminoglycan, and modifications or variants thereof. Examples of proteoglycans include chondroitin sulfate proteoglycan, heparan sulfate proteoglycan, keratan sulfate proteoglycan, dermatan sulfate proteoglycan, and the like. Examples of glycosaminoglycans include hyaluronic acid, chondroitin 4-sulfate, chondroitin 6-sulfate, dermatan sulfate, keratan sulfate, heparan sulfate, heparin, and the like. The mixture prepared in step (A) may be mixed with only one extracellular matrix component, or two or more extracellular matrix components in combination. In the production of the cell structure according to the present embodiment, it is preferable to use one or more members selected from the group consisting of collagen, laminin, and fibronectin; and it is particularly preferable to use collagen. The amount of extracellular matrix component mixed in the solution for cell suspension is not particularly limited, as long as it does not adversely affect the cell growth and the production of the cell structure. For example, the concentration of the extracellular matrix component in the solution for cell suspension may be more than 0 mg/mL, preferably 0.010 mg/mL or more, more preferably 0.020 mg/mL or more, even more preferably 0.025 mg/mL or more, and still even more preferably 0.05 mg/mL or more. Further, the concentration of the extracellular matrix component in the solution for cell suspension is preferably less than 1.0 mg/mL, more preferably 0.75 mg/mL or less, even more preferably 0.5 mg/mL or less, still even more preferably 0.25 mg/mL or less, and particularly preferably 0.1 mg/mL or less.

The mixing ratio of the polyelectrolyte to the extracellular matrix component in the solution for cell suspension is in the range of 1:2 to 2:1. In the production of the cell structure according to the present embodiment, the mixing ratio of the polyelectrolyte to the extracellular matrix component is preferably in the range of 1:1.5 to 1.5:1, and more preferably 1:1.

After steps (A) and (B) are performed at least once, step (C) is performed. Specifically, the mixture prepared in step (A) is seeded as step (B), then step (A) is performed using the cells to be laminated next to prepare a mixture, and the mixture is then seeded in the cell aggregate obtained in previous step (B) as step (B). After this operation is repeated as many times as necessary, step (C) can be performed, thereby producing a cell structure with a sufficient thickness. In step (A), the cell composition of the mixture newly seeded on the cell aggregate obtained in step (B) may be the same as or different from the cell composition that constitutes the already produced cell aggregate. Further, steps (A) may be performed simultaneously.

For example, step (A) is first performed to prepare a mixture containing only fibroblasts as cells, and then step (B) is performed to obtain a cell aggregate composed of 10 fibroblast layers in a cell culture container. Subsequently, step (A) is performed to prepare a mixture containing only migratory cells as cells, and then step (B) is performed to laminate one migratory cell layer on the 10 fibroblast layers in the cell culture container. Further, step (A) is performed to prepare a mixture containing only fibroblasts as cells, and then step (B) is performed to laminate 10 fibroblast layers on the migratory cell layer in the cell culture container. This enables the production of a cell structure composed of a stromal cell layer in which 10 fibroblast layers, one migratory cell layer, and 10 fibroblast layers are sequentially laminated in layers for each cell type. The thickness of cell layers laminated in step (C) can be adjusted by controlling the number of cells seeded in step (B). As the number of cells seeded in step (B) increases, the number of cell layers laminated in step (C) increases. Furthermore, a mixture containing all of the fibroblasts for 20 fibroblast layers, the migratory cells for 1 migratory cell layer, is prepared in step (A), and then steps (B) and (C) are performed, thereby producing a cell structure having a thickness corresponding to the 21 layers, in which the migratory cells are scattered throughout the structure. As for cell structures with a vascular network formed in the stromal cell layer, a cell structure with a vascular network formed only in a specific cell layer, and a cell structure in which a vascular network is scattered throughout the entire stromal cell layer of the cell structure, can be produced in the same manner. Furthermore, step (A) is performed to prepare a mixture containing all the cells constituting the stromal cell layer, that is, all of the fibroblasts, vascular endothelial cells, and migratory cells, and then steps (b) and (c) are performed to thereby produce a cell structure in which the migratory cells and a vascular network structure are both independently scattered throughout the structure.

After step (A), step (A'-1) may be performed to remove the liquid part from the obtained mixture to obtain a cell aggregate, step (A'-2) may be performed to suspend the cell aggregate in a solution, and in place of step (B), step (B') may be performed to precipitate cells from the obtained suspension to form a cell precipitate on the substrate.

In step (A), the cells and the solution for cell suspension may be mixed in a suitable container, such as a dish, tube, flask, bottle, or plate, or on the substrate used in step (B). Suspension in step (A'-2) may also be carried out in a suitable container, such as a dish, tube, flask, bottle, or plate, or on the substrate used in step (B').

As a means for removing the liquid part in step (A' -1), methods known to a person skilled in the art can be used. For example, the liquid part may be removed by centrifugation or filtration. The centrifugation conditions are not particularly limited, as long as they do not adversely affect the cell growth and the formation of cell aggregates. For example, a microtube containing the mixture is subjected to centrifugation at room temperature and 400×g for 1 minute to separate a liquid part and at least one cell aggregate, thereby removing the liquid part. Alternatively, the liquid part may be removed after the cells are collected by spontaneous precipitation.

The solution used in step (A'-2) is not particularly limited, as long as it does not adversely affect the cell growth and the formation of cell aggregates. For example, a cell culture medium or buffer solution suitable for the cells to be used is used.

Examples of the substrate used in step (B) or (B') include a culture container for use in culturing cells. The culture container may be a container of material and shape which are commonly used for culturing cells and microorganisms. Examples of the material of the culture container include, but are not limited to, glass, stainless steel, plastic, and the like. Examples of the culture container include, but are not limited to, dishes, tubes, flasks, bottles, plates, and the like. The substrate may be, for example, a material that allows liquid to pass through without allowing cells in the liquid to pass through.

The substrate used in step (B) is preferably a permeable membrane. Examples of containers having a permeable membrane include, but are not limited to, cell culture inserts, such as Transwell (registered trademark) insert, Netwell (registered trademark) insert, Falcon (registered trademark) cell culture insert, and Millicell (registered trademark) cell culture insert.

As a means for collecting the cells in step (B) or (B'), methods known to a person skilled in the art can be used. For example, the cells may be collected by centrifugation, magnetic separation, or filtration. The centrifugation conditions are not particularly limited, as long as they do not adversely affect the cell growth. For example, the cells may be collected by seeding the mixture or suspension in a cell culture insert, followed by centrifugation at 10°C and 400×g for 1 minute. Alternatively, the cells may be collected by spontaneous precipitation. In step (B'), for example, a cell precipitate may be formed on the substrate by removing the liquid part from the suspension by centrifugation or filtration. Alternatively, the cell precipitate may be formed on the substrate by spontaneous precipitation. The cell aggregate in step (B) or the cell precipitate in step (B') may be in the form of laminae.

In step (C), the cells can be cultured under culture conditions suitable for the cells to be cultured. A person skilled in the art can select a suitable medium depending on the cell type and desired function. Various conditions, such as culture temperature and culture time, can also be easily determined by a person skilled in the art.

For example, first, steps (A) and (B) are performed at least once using the cells that constitute the stromal cell layer, and then step (C) is performed to produce a stromal cell layer. Thereafter, the produced cell structure (i.e., stromal cell layer) is subjected to steps (A) and (B) at least once using the cells that constitute the migratory cell-free cell layer, and then step (C) is performed to produce a migratory cell-free cell layer on the top surface of the stromal cell layer. Thus, the cell structure according to the present embodiment can be produced.

When a vascular network structure is formed in the stromal cell layer, steps (A) and (B) are performed at least once using the cells that constitute the stromal cell layer containing vascular endothelial cells, and then the cells are cultured for a predetermined period of time, generally about 1 to 3 days, in step (C). During this culture period, a vascular network is formed within the stromal cell layer. Thereafter, the cell structure composed of the stromal cell layer is subjected to steps (A) and (B) at least once using the cells that constitute the migratory cell-free cell layer, and then step (C) is performed to form a migratory cell-free cell layer on the top surface of the stromal cell layer. Thus, among the cell structures according to the present embodiment, a cell structure with a vascular network structure formed in the stromal cell layer can be produced. When the stromal cell layer is cultured for a predetermined period of time before the migratory cell-free cell layer is laminated to form a vascular network, the migratory cells in the stromal cell layer migrate randomly during the culture period. Therefore, in the case of this method, even when migratory cells are present only in a specific cell layer before step (C) is performed, the migratory cells are present randomly in the stromal cell layer at the time of laminating the migratory cell-free cell layer.

The size and shape of the cell structure according to the present embodiment are not particularly limited, as long as the migration ability of migratory cells can be evaluated. The migration ability of migratory cells is evaluated based on the mobility in the cell structure; however, because the mobility of migratory cells can be more easily measured, the thickness of the cell structure is preferably 5 µm or more, more preferably 10 µm or more, even more preferably 50 µm or more, and still even more preferably 100 µm or more. The thickness of the cell structure is preferably 500 µm or less, more preferably 400 µm or less, and even more preferably 300 µm or less. The upper and lower limit values of the thickness of the cell structure can be combined in any way. For example, the thickness of the cell structure is preferably 5 µm or more and 500 µm or less, more preferably 10 µm or more and 400 µm or less, even more preferably 50 µm or more and 300 µm or less, and still even more preferably 100 µm or more and 300 µm or less. The number of cell layers in the stromal cell layer of the cell structure according to the present embodiment is preferably approximately in the range of 2 to 60, more preferably approximately in the range of 5 to 60, and even more preferably approximately in the range of 10 to 60. The number of cell layers in the migratory cell-free cell layer of the cell structure according to the present embodiment may be 1, but is preferably 2 or more, and more preferably 5 or more, because the migration ability of migratory cells can be evaluated more accurately.

The number of cell layers in the cell structure can be measured based on a cross-sectional image of a section of the cell structure, which is observed at a magnification at which a cell nucleus can be recognized, that is, at which the entire thickness of the nuclear-stained section is included in the field of view. In the section image, when observed, the number of layers composed of a group of cells and stroma that exist in a direction perpendicular to the thickness direction and whose cell nuclei do not overlap in the thickness direction is counted. This counted number of layers is the number of cell layers in the cell structure.

Further, in the cell structure according to the present embodiment, the theoretical number of cell layers can also be taken as the number of cell layers in the cell structure. Here, the theoretical number of cell layers that constitute the cell structure can be measured by dividing the total number of cells that constitute the cell structure by the number of cells per layer (the number of cells required to constitute one layer). The number of cells per layer (Ns) can be determined by measuring the number of cell layers (L₁) in a cell structure obtained by using the same type of cells on the same type of substrate, and dividing the total number of cells (N_{A}) that constitute the cell structure by L₁ (N_{S}=N_{A}/L₁).

### <Method for evaluating the migration ability of migratory cells >

The method for evaluating the migration ability of migratory cells according to one embodiment of the present invention is a method for evaluating the migration ability of migratory cells, comprising culturing the cell structure according to the present embodiment, and evaluating the migration ability using the mobility of migratory cells in the cell structure as an indicator. That is, the evaluation method according to the present embodiment has the following steps:
a culture step of culturing a cell structure having a stromal cell layer containing migratory cells and stromal cells, and a migratory cell-free cell layer containing no migratory cells on a top surface of the stromal cell layer; and
an evaluation step of evaluating the migration ability of the migratory cells based on the mobility of the migratory cells in the cell structure after the culture step.

The mobility of migratory cells is the ratio of the height Hm of the migratory cells in the cell structure to the height Hs of the cell structure (Hm/Hs). Hs and Hm both can be measured based on a cross-sectional image of a section of the cell structure, which is observed at a magnification at which a cell nucleus can be recognized, that is, at which the entire thickness of the nuclear-stained section is included in the field of view. In the section image, first, a migratory cell whose mobility is to be measured is determined, an intersection between a perpendicular line drawn from the migratory cell down to the bottom of the cell structure and the bottom of the cell structure is regarded as a point Pb, and an intersection between a straight line obtained by extending the perpendicular line to the top surface side of the cell structure and the top surface of the cell structure is regarded as a point Pt. The height Hm of the migratory cell in the cell structure is the distance between the point Pb and the migratory cell, and the height Hs of the cell structure is the distance between the points Pb and Pt. It is also preferable that a section image is obtained after the section of the cell structure is subjected to a staining process for migratory cells in advance in addition to nuclear staining.

The culture time in the culture step is not particularly limited, as long as the migratory cells within the cell structure have enough time to migrate within the cell structure. For example, the culture time is preferably 12 hours or more, more preferably 1 day or more, and even more preferably 1 to 3 days. The culture medium, culture temperature, and other culture conditions can be similar to those for culturing stromal cells in general.

When the second cell structure is used, regardless of the level of the migration ability of migratory cells, the migratory cells within the cell structure migrate in random directions inside the cell structure. Accordingly, the variation in the mobility of each migratory cell within the cell structure is large.

When the first cell structure is used, the migratory cells are attracted to the migration target cells on the top surface of the cell structure. Therefore, the variation in the mobility of each migratory cell within the cell structure is smaller than when using the second cell structure. Moreover, when the migratory cells have sufficient migration ability, almost all of the migratory cells within the cell structure migrate toward the migration target cells on the top surface of the cell structure, regardless of the distance between the migratory cells and the migration target cells at the start of culture. Therefore, as the migratory cells have higher migration ability, the mobility of the migratory cells is closer to the maximum value of 100%. If the migratory cells have low migration ability, the migratory cells within the cell structure migrate more randomly in the stromal cell layer; thus, the average mobility of the migratory cells within the cell structure will only be about the same as the average mobility of the migratory cells within the second cell structure. That is, as the average mobility of the migratory cells within the cell structure is closer to 100%, the migration ability of the migratory cells is evaluated as higher, and as the average mobility is lower, the migration ability of the migratory cells is evaluated as lower.

The above explanation explains an example in which a migratory cell-free cell layer containing migration target cells is located on the top surface of the cell structure. When the migratory cell-free cell layer is not located on the top surface of the cell structure, if the migratory cells migrate toward the migration target cells, the mobility will be lower than 100%. Even in that case, when the migratory cells have sufficient migration ability, almost all of the migratory cells within the cell structure migrate toward the migration target cells, regardless of the distance between the migratory cells and the migration target cells at the start of culture. Therefore, as the migratory cells have higher migration ability, the mobility of the migratory cells becomes a numerical value corresponding to the position of the migratory cell-free cell layer, and the variation in the mobility of the migratory cells becomes less.

When the migratory cells are CTLs, the evaluation method according to the present embodiment can evaluate, in addition to the migration ability of migratory cells, the cytotoxicity of the migratory cells against the migration target cells. The cytotoxicity against the migration target cells is the ratio of the number of dead migration target cells to all migration target cells in the cell structure. The cytotoxicity against the migration target cells may be determined by subtracting, from 1, the ratio of the number of live migration target cells to the number of all migration target cells in the cell structure.

When the first cell structure is used, if the average mobility is high and the cytotoxicity against migration target cells is also high, these CTLs can easily reach target cancer cells in vivo and have high attack power, so they can be evaluated as migratory cells that are highly effective in cancer immunity etc. On the other hand, if the cytotoxicity against migration target cells is low while the average mobility is high, the CTLs are evaluated to have high migration ability but low cytotoxicity. When the second cell structure is used, if the cytotoxicity against migration target cells is high, the CTLs are evaluated to be highly toxic to cells other than the target cells.

### Examples

The present invention will be more specifically described in greater detail below by way of examples. However, the following examples should not limit the scope of the present invention.

### [Example 1]

Five types of cell structures, which were different in terms of the presence or absence of CTLs, the locations of cancer cells and CTLs in the structure, and the presence or absence of a blood vessel network structure, were produced, and the mobility and cytotoxicity of CTLs were examined.

The vascular endothelial cells used were human umbilical vein endothelial cells labeled with fluorescent protein RFP (also referred to as RFP-HUVEC) (model number: cAP-0001RFP, produced by ANGIO-PROTEOMIE), the stromal cells used were human neonatal dermal fibroblasts (also referred to as NHDF) (model number: CC-2509, produced by Lonza), the migratory cells used were CTLs (provided by the Sapporo Medical University, NPL 7), the CTL-specific cancer cells (i.e., cancer cells that bind to CTLs via HLA/neoantigen AKF9, and that are toxic or attractive) used were cells in which fluorescent protein GFP was expressed in β2m-expressing human colon adenocarcinoma-derived cultured cell line HCT15 (HCT15/β2m) (provided by the Sapporo Medical University) (hereinafter, "HCT15 cells"), and the non-CTL-specific cancer cells (i.e., cancer cells that do not bind to CTLs via HLA/neoantigen AKF9, and that are neither toxic nor attractive nor repellent) used were human colon adenocarcinoma-derived cultured cell line Colo320 (provided by the Sapporo Medical University).

In the production of the cell structures, the polyelectrolyte used was a heparin sodium salt (model number: H3149-100KU, produced by Sigma), and the extracellular matrix components used were collagen (Collagen Type I, Bovine Skin, Acid Soluble) (model number: ASC-1-100-100, produced by NIP) and fibronectin (model number: F4759-5MG, produced by Sigma). The general-purpose medium used was DMEM medium (model number: 043-30085, produced by Wako Pure Chemical Industries, Ltd.) containing 10% FBS and 1% antibiotic, the endothelial cell medium used was EGM-2MV BulletKit medium (model number: CC-3202, produced by Lonza), and the CTL medium used was AIM-V medium (model number: 0870112DK, produced by Gibco) containing 1% HEPES, 55 mM 2-mercaptoethanol, and 1% antibiotic. The antibiotic used was Penicillin-Streptomycin (also referred to as P/S) (model number: 168-23191, produced by Wako Pure Chemical Industries, Ltd.). The culture container used was a Transwell culture insert (model number: 3470, produced by Corning). The cell fixative used was a 10% formalin buffer solution (model number: 062-01661, produced by Wako Pure Chemical Industries, Ltd.).

### (1) Cell structure A

A cell structure A was produced containing a cancer cell layer held between stromal cell layers and a blood vessel network structure formed throughout the cell structure, and then cultured in a medium containing CTLs.

First, as a solution for cell suspension, a 0.1 mg/mL heparin/50 mM tris-hydrochloric acid buffer (pH: 7.4) solution and a 0.1 mg/mL collagen/acetic acid solution (pH: 3.7) were mixed in equal amounts to prepare a solution (i.e., final concentrations of collagen and heparin: 0.05 mg/mL). Then, a cell population containing a mixture of 1×10⁶ cells of NHDF and 1.5×10⁴ cells of RFP-HUVEC was suspended in the solution for cell suspension to prepare a cell mixture. Then, the cell mixture was centrifuged at room temperature at 1000×g for 2 minutes, the supernatant was removed, and 300 µL of general-purpose medium was then added to prepare a cell suspension.

Then, 1 mL of general-purpose medium was added to the outside of the culture container, 300 µL of the cell suspension was added into a culture container insert coated with 0.1 mg/mL fibronectin in advance to seed the cells, followed by centrifugation at room temperature at 400×g for 2 minutes, and the cells were statically cultured in a CO₂ incubator (37°C, 5% CO₂) for 2 hours. Thereafter, 1 mL of general-purpose medium was added to the outside of the culture container, and the cells were statically cultured in a CO₂ incubator (37°C, 5% CO₂) overnight. A stromal cell layer was formed in this way.

Then, the medium outside the culture container in which a stromal cell layer was formed was removed, and 1 mL of general-purpose medium was added. Thereafter, the medium in the insert of the culture container was removed, and 1×10³ cancer cells suspended in 100 µL of general-purpose medium were seeded and then statically cultured in a CO₂ incubator (37°C, 5% CO₂) for 2 hours. A cancer cell layer was formed on the top surface of the stromal cell layer in this way.

Thereafter, a cell population containing a mixture of 1×10⁶ cells of NHDF and 1.5×10⁴ cells of RFP-HUVEC was suspended in the solution for cell suspension to prepare a cell mixture, the cell mixture was centrifuged at room temperature at 1000×g for 2 minutes to remove the supernatant, and 200 µL of general-purpose medium was then added to prepare a cell suspension. 200 µL of the cell suspension was added into the insert of the culture container in which a cancer cell layer was formed to seed the cells, followed by centrifugation at room temperature at 400×g for 2 minutes, and the cells were then statically cultured in a CO₂ incubator (37°C, 5% CO₂) for 2 hours. Thereafter, 1 mL of general-purpose medium was added to the outside of the culture container, and the cells were statically cultured in a CO₂ incubator (37°C, 5% CO₂) for 2 hours. Thereafter, 1 mL of general-purpose medium was added to the outside of the culture container, and the cells were statically cultured in a CO₂ incubator (37°C, 5% CO₂) for 3 days. The cell structure A with a cancer cell layer held between stromal cell layers was produced in this way.

The cell structure A was cultured in a culture medium containing CTLs. Specifically, the medium outside the culture container was removed, 1 mL of CTL medium was added, then the medium in the culture container insert was removed, and 2.5×10³ CTLs suspended in 300 µL of CTL medium were added into the culture container insert and statically cultured in a CO₂ incubator (37°C, 5% CO₂) for 3 days.

After the cultured cell structure A was washed twice with PBS, 300 µL of cell fixative was added into the culture container insert and allowed to stand at room temperature for 15 minutes or more, followed by washing three times with PBS.

### (2) Cell structure B

A cell structure B was produced having a stromal cell layer in which CTLs were evenly distributed throughout the layer, and a cancer cell layer on the top surface of the stromal cell layer.

First, a cell population containing a mixture of 2×10⁶ cells of NHDF and 3×10⁴ cells of RFP-HUVEC was suspended in the solution for cell suspension to prepare a cell mixture. Then, the cell mixture was centrifuged at room temperature at 1000×g for 2 minutes, the supernatant was removed, 300 µL of general-purpose medium was then added for suspension, and 1×10⁴ CTLs were added to prepare a cell suspension.

Then, 1 mL of general-purpose medium was added to the outside of the culture container, 300 µL of the cell suspension was added into a culture container insert coated with 0.1 mg/mL fibronectin in advance to seed the cells, followed by centrifugation at room temperature at 400×g for 2 minutes, and the cells were statically cultured in a CO₂ incubator (37°C, 5% CO₂) for 2 hours. Thereafter, 1 mL of general-purpose medium was added to the outside of the culture container, and the cells were statically cultured in a CO₂ incubator (37°C, 5% CO₂) overnight. A stromal cell layer containing CTLs was formed in this way.

Then, the medium outside the culture container in which a stromal cell layer was formed was removed, and 1 mL of general-purpose medium was added. Thereafter, the medium in the insert of the culture container was removed, and 1×10⁴ or 3×10⁴ cancer cells suspended in 300 µL of general-purpose medium were seeded and then statically cultured in a CO₂ incubator (37°C, 5% CO₂) for 2 hours. Thereafter, 1 mL of general-purpose medium was added to the outside of the culture container, and the cells were statically cultured in a CO₂ incubator (37°C, 5% CO₂) for 1 day or 3 days. The cell structure B with a cancer cell layer on the top surface of a stromal cell layer was produced in this way.

After the cultured cell structure B was washed twice with PBS, 300 µL of cell fixative was added into the culture container insert and allowed to stand at room temperature for 15 minutes or more, followed by washing three times with PBS.

### (3) Cell structure C

A cell structure C was produced having a stromal cell layer formed such that CTLs were present in a specific cell layer located in the middle of the stromal cell layer, and a cancer cell layer on the top surface of the stromal cell layer.

First, a cell population containing a mixture of 1×10⁶ cells of NHDF and 1.5×10⁴ cells of RFP-HUVEC was suspended in the solution for cell suspension to prepare a cell mixture. Then, the cell mixture was centrifuged at room temperature at 1000×g for 2 minutes, the supernatant was removed, and 200 µL of general-purpose medium was then added to prepare a cell suspension.

Then, 1 mL of general-purpose medium was added to the outside of the culture container, 200 µL of the cell suspension was added into a culture container insert coated with 0.1 mg/mL fibronectin in advance to seed the cells, followed by centrifugation at room temperature at 400×g for 2 minutes, and the cells were then statically cultured in a CO₂ incubator (37°C, 5% CO₂) for 2 hours. A stromal cell layer was formed in this way.

Then, 1×10⁴ CTLs suspended in 100 µL of general-purpose medium were seeded in the insert of the culture container in which a stromal cell layer was formed, and then statically cultured in a CO₂ incubator (37°C, 5% CO₂) for 2 hours. A CTL layer was formed on the top surface of the stromal cell layer in this way.

Thereafter, a cell population containing a mixture of 1×10⁶ cells of NHDF and 1.5×10⁴ cells of RFP-HUVEC was suspended in the solution for cell suspension to prepare a cell mixture, the cell mixture was centrifuged at room temperature at 1000×g for 2 minutes to remove the supernatant, and 200 µL of general-purpose medium was then added to prepare a cell suspension. 200 µL of the cell suspension was added into the insert of the culture container in which a CTL layer was formed to seed the cells, followed by centrifugation at room temperature at 400×g for 2 minutes, and the cells were then statically cultured in a CO₂ incubator (37°C, 5% CO₂) for 2 hours. Thereafter, 1 mL of general-purpose medium was added to the outside of the culture container, and the cells were statically cultured in a CO₂ incubator (37°C, 5% CO₂) for 2 hours. Thereafter, 1 mL of general-purpose medium was added to the outside of the culture container, and the cells were statically cultured in a CO₂ incubator (37°C, 5% CO₂) overnight. A stromal cell layer with a CTL layer in the middle was produced in this way.

Then, the cell structure C having a cancer cell layer formed on the top surface of the stromal cell layer was produced in the same manner as in (2) above and fixed.

### (4) Cell structure D

A cell structure D was produced having a stromal cell layer formed such that CTLs were present in the bottom cell layer of the stromal cell layer, and a cancer cell layer on the top surface of the stromal cell layer.

First, 1 mL of general-purpose medium was added to the outside of the culture container, 1×10⁴ CTLs suspended in 100 µL of general-purpose medium were seeded in a culture container insert coated with 0.1 mg/mL fibronectin in advance, and then statically cultured in a CO₂ incubator (37°C, 5% CO₂) for 2 hours. A CTL layer was formed in this way.

Further, a cell population containing a mixture of 2×10⁶ cells of NHDF and 3×10⁴ cells of RFP-HUVEC was suspended in the solution for cell suspension to prepare a cell mixture. Then, the cell mixture was centrifuged at room temperature at 1000×g for 2 minutes, the supernatant was removed, and 200 µL of general-purpose medium was then added to prepare a cell suspension.

Then, 200 µL of the cell suspension was added into the insert of the culture container in which a CTL layer was formed to seed the cells, followed by centrifugation at room temperature at 400×g for 2 minutes, and the cells were then statically cultured in a CO₂ incubator (37°C, 5% CO₂) for 2 hours. Thereafter, 1 mL of general-purpose medium was added to the outside of the culture container, and the cells were statically cultured in a CO₂ incubator (37°C, 5% CO₂) for 2 hours. Thereafter, 1 mL of general-purpose medium was added to the outside of the culture container, and the cells were statically cultured in a CO₂ incubator (37°C, 5% CO₂) overnight. A stromal cell layer with a CTL layer in the bottom was produced in this way.

Then, the cell structure D having a cancer cell layer formed on the top surface of the stromal cell layer was produced in the same manner as in (2) above and fixed.

### (5) Cell structure E

A cell structure E was produced having a stromal cell layer in which CTLs were evenly distributed throughout the layer and a blood vessel network was formed throughout the layer, and a cancer cell layer on the top surface of the stromal cell layer.

First, a cell population containing a mixture of 2×10⁶ cells of NHDF and 3×10⁴ cells of RFP-HUVEC was suspended in the solution for cell suspension to prepare a cell mixture. Then, the cell mixture was centrifuged at room temperature at 1000×g for 2 minutes, the supernatant was removed, 300 µL of general-purpose medium was then added for suspension, and 1×10⁴ CTLs were added to prepare a cell suspension.

Then, 1 mL of general-purpose medium was added to the outside of the culture container, 300 µL of the cell suspension was added into a culture container insert coated with 0.1 mg/mL fibronectin in advance to seed the cells, followed by centrifugation at room temperature at 400×g for 2 minutes, and the cells were statically cultured in a CO₂ incubator (37°C, 5% CO₂) for 2 hours. Thereafter, 1 mL of general-purpose medium was added to the outside of the culture container, and the cells were statically cultured in a CO₂ incubator (37°C, 5% CO₂) for 4 days. A stromal cell layer in which CTLs were evenly distributed and a blood vessel network was formed throughout the layer was formed in this way.

Then, the cell structure E having a cancer cell layer formed on the top surface of the stromal cell layer was produced in the same manner as in (2) above and fixed.

### (6) Control cell structure

Regarding the cell structures B to E, cell structures consisting of a stromal cell layer before a cancer cell layer was formed on the top surface thereof were regarded as control cell structures B to E and fixed in the same manner.

### (7) Production of section specimen of cell structure

First, each cell structure was embedded using a low toxicity solvent (product name: G-Nox, produced by GenoStaff) and a paraffin embedding device (product name: CT-Pro20, produced by GenoStaff), and then sliced thinly parallel to the thickness direction of the cell structure to produce a thin layer section with a thickness of 6 µL.

The obtained thin layer section was subjected to heat treatment (microwave, EDTA buffer, pH: 9) to activate the antigen, followed by immunohistochemical staining (IHC) with 0.2 µg/mL anti-CD8 antibody (also referred to as CD8 mouse mAb) (model number: #M7103, produced by Dako).

### (8) Measurement of mobility (%)

The IHC-completed section specimen (glass slide) of each cell structure was observed with an optical microscope (product name: MX51, produced by Olympus Corporation) at a magnification at which the entire thickness of the section was included in the field of view, and an image was obtained. All CTLs in the obtained cell staining image were subjected to image analysis to measured Hm/Hs.

Specifically, in the section image, for each CTL, an intersection between a perpendicular line drawn from the CTL to the bottom of the cell structure and the bottom of the cell structure was regarded as a point Pb, an intersection between a straight line obtained by extending the perpendicular line to the top surface side of the cell structure and the top surface of the cell structure was regarded as a point Pt, and the distance between the CTL and the point Pb (Hm) and the distance between the points Pt and Pb (Hs) were measured. Since CTLs are CD8-positive cells, the CTLs in the cell structure are CD8-positive areas (areas immunostained with anti-CD8 antibody) in the image. The obtained Hs was divided by Hm to determine the mobility Hm/Hs.

### (9) Measurement of relative viability (%)

Each cell structure was photographed with a microscope system (product name "Operetta CLS," produced by PerkinElmer) to include the entire inside of the culture container insert, and the obtained image was analyzed to calculate the percentage of cancer cells occupying the insert.

For the cell structure containing HCT15 cells, GFP expressing in HCT15 cells was detected to calculate the percentage of cancer cells (confluency).

For the cell structure containing Colo320 cells, cancer cells were detected by immunostaining (IF/ICC) using anti-EpCAM antibody (D4K8R, rabbit, monoclonal, produced by Cell Signaling Technology) as the primary antibody and Alexa Flour 647 antibody (A21236, produced by Thermo Fisher Scientific) as the secondary antibody, and the percentage thereof (confluency) was calculated.

The percentage of cancer cells in each cell structure was determined as a relative viability (%) when the percentage of cancer cells in the corresponding CTL-free cell structure A was taken as 100%. The cytotoxicity (%) is 100 - relative viability (%).

Tables 1 and 2 show the results of the mobility (%) of CTLs and the relative viability (%) of cancer cells measured for the cell structures A to E and the control cell structures B to E. Table 1 shows the results of cell structures cultured for 1 day after a cancer cell layer was laminated, and Table 2 shows the results of cell structures cultured for 3 days after a cancer cell layer was laminated. In the tables, the "Cancer cells" column indicates the type of cancer cells used in the cell structure, and the "Number of cells seeded" column indicates the number of cancer cells constituting the cancer cell layer of the cell structure. The cell structures with "w/o" in the "Cancer cells" column and "0" in the "Number of cells seeded" column show the results of the control cell structures, in which a cancer cell layer was not laminated.

**[Table 1]**

| Cell structure | | | Relative viability [%] | CTL mobility [%] | | | | Number of CTLs |
|---|---|---|---|---|---|---|---|---|
| | Cancer cells | Number of cells seeded | | Average value | Median value | Maximum value | Minimum value | |
| B | HCT15 | 1×10³ | 40.5 | 92.93 | 96.82 | 99.50 | 22.40 | 22 |
| B | HCT15 | 3×10⁴ | 49.19 | 89.98 | 97.33 | 98.71 | 49.28 | 11 |
| C | HCT15 | 1×10³ | 39.03 | 92.52 | 94.8 | 99.16 | 78.26 | 12 |
| C | HCT15 | 3×10⁴ | 83.72 | 87.26 | 97.95 | 99.36 | 27.35 | 18 |
| C | Colo320 | 1×10⁴ | 154.887 | 47.54 | 34.94 | 99.04 | 5.97 | 27 |
| C | w/o | 0 | - | 51.19 | 50.84 | 97.10 | 1.47 | 13 |
| C | HCT15 | 1×10³ | 54.12 | 90.06 | 93.38 | 97.45 | 49.39 | 17 |
| C | HCT15 | 3×10⁴ | 82.72 | 65.60 | 96.66 | 98.84 | 90.89 | 14 |
| C | Colo320 | 1×10⁴ | 135.251 | 74.13 | 84.98 | 97.93 | 0.64 | 25 |
| C | w/o | 0 | - | 59.74 | 68.46 | 98.31 | 0.00 | 41 |
| D | HCT15 | 3×10⁴ | 62.87 | 86.05 | 93.16 | 96.61 | 36.19 | 7 |
| D | Colo320 | 1×10⁴ | No data | 53.07 | 52.98 | 97.78 | 3.26 | 14 |
| D | w/o | 0 | - | 66.52 | 85.28 | 98.74 | 0.00 | 11 |

**[Table 2]**

| Cell structure | | | Relative viability [%] | CTL mobility [%] | | | | Number of CTLs |
|---|---|---|---|---|---|---|---|---|
| | Cancer cells | Number of cells seeded | | Average value | Median value | Maximum value | Minimum value | |
| A | HCT15 | 3×10⁴ | 57.16 | 49.69 | 50.90 | 94.27 | 0.00 | 17 |
| A | Colo320 | 1×10⁴ | 90.71 | 48.90 | 44.44 | 78.65 | 28.07 | 4 |
| A | w/o | 0 | - | 36.17 | 31.66 | 63.54 | 1.26 | 5 |
| B | HCT15 | 1×10³ | 5.52 | 88.15 | 94.21 | 100.00 | 40.66 | 15 |
| B | HCT15 | 3×10⁴ | 29.89 | 85.35 | 93.95 | 100.00 | 0.00 | 28 |
| C | HCT15 | 1×10³ | 8.73 | 83.38 | 91.62 | 98.57 | 30.19 | 15 |
| C | HCT15 | 3×10⁴ | 34.52 | 69.65 | 82.87 | 96.32 | 0.00 | 14 |
| C | Colo320 | 1×10⁴ | 113.27 | 61.42 | 74.92 | 97.49 | 1.54 | 25 |
| C | w/o | 0 | - | 56.56 | 65.39 | 97.71 | 0.00 | 22 |
| C | HCT15 | 1×10³ | 9.73 | 78.94 | 90.10 | 97.49 | 26.84 | 20 |
| C | HCT15 | 3×10⁴ | 28.84 | 93.66 | 94.70 | 97.29 | 86.34 | 14 |
| C | Colo320 | 1×10⁴ | 111.49 | 61.71 | 79.22 | 99.44 | 0.00 | 21 |
| C | w/o | 0 | - | 43.78 | 37.78 | 95.01 | 0.00 | 13 |

As shown in Tables 1 and 2, in any of the cell structures B to E, the mobility of most CTLs in the cell structures having a cancer cell layer of CTL-specific cancer cells HCT15 was around 100%, whereas the mobility in the control cell structures was widely distributed from 0 to 100%. Further, in the cell structures having a cancer cell layer of non-CTL-specific cancer cells Colo320, the mobility was widely distributed from 0 to 100%, as in the control cell structures, and a significant difference in mobility was observed compared to the cell structures containing CTL-specific cancer cells. On the other hand, in the cell structures with a stromal cell layer containing CTLs and a cancer cell layer formed on the top surface thereof, the position of CTLs in the stromal cell layer and the presence or absence of a blood vessel network structure had no significant effect on the mobility of CTLs.

Further, as shown in Table 2, in the cell structures A, which did not contain CTLs, there was no difference in the distribution of the mobility of CTLs between the control cell structure, the cell structure containing CTL-specific cancer cells, and the cell structure containing non-CTL-specific cancer cells. It was confirmed that with these cell structures, differences in the migration ability of CTLs could not be evaluated based on the mobility of CTLs.

In addition, Fig. 1 shows the relationship between the CTL mobility (%) of the cell structures C and A, and the relative viability (%) of cancer cells. As shown in the figure, for the cell structures C, the plot was clearly divided into a group of CTL-specific cancer cell-containing cell structures and a group of non-CTL-specific cancer cell-containing cell structures, and the correlation between the cytotoxicity of CTLs against cancer cells and the mobility of CTLs could be evaluated. Further, looking at the plot by evaluation date, it was confirmed that the plot was shifted to the left from the cell structure after 1 day of culture to the cell structure after 3 days of culture. From the above results, it was predicted that cancer cells would not be killed when CTLs and the cancer cells were separated, and that the cancer cells would be killed by CTLs only when CTLs migrated to the cancer cells and both cells approached sufficiently closely. The plot showed changes in accordance with the prediction of changes over time, and the model using the cell structures according to the present embodiment was considered to be capable of evaluating the correlation between the mobility and cytotoxicity of CTLs.

### [Industrial Applicability]

In the cell structure according to the present embodiment, migratory cells, such as CTLs, are contained inside the cell structure composed of stromal cells, and the migratory cells migrate within the cell layer of the stromal cells. Therefore, the method for evaluating the migration ability of migratory cells according to the present embodiment can more accurately evaluate the migration ability of migratory cells inside the interstitial tissue by using this cell structure.

## Claims

1. A method for evaluating the migration ability of migratory cells, comprising:
a culture step of culturing a cell structure having a stromal cell layer containing migratory cells and stromal cells, and a migratory cell-free cell layer containing no migratory cells on a top surface of the stromal cell layer; and
an evaluation step of evaluating the migration ability of the migratory cells based on the mobility of the migratory cells in the cell structure after the culture step,
the mobility of the migratory cells being a ratio of a height Hm of the migratory cells in the cell structure to a height Hs of the cell structure,
the height Hm of the migratory cells in the cell structure being the distance between a point Pb and the migratory cells, the point Pb being an intersection where a perpendicular line down from the migratory cells to a bottom of the cell structure intersects the bottom of the cell structure, and
the height Hs of the cell structure being a distance between the point Pb and a point Pt, the point Pt being an intersection where a straight line obtained by extending the perpendicular line to a top surface of the cell structure intersects the top surface of the cell structure.

2. The method for evaluating the migration ability of migratory cells according to claim 1, wherein
the stromal cells are at least one selected from the group consisting of fibroblasts, immune cells other than the migratory cells, and mast cells.

3. The method for evaluating the migration ability of migratory cells according to claim 1 or 2, wherein
the stromal cell layer contains vascular endothelial cells.

4. The method for evaluating the migration ability of migratory cells according to claim 1 or 2, wherein
the migratory cells are immune cells.

5. The method for evaluating the migration ability of migratory cells according to claim 4, wherein
the migratory cells are cytotoxic T cells.

6. The method for evaluating the migration ability of migratory cells according to claim 1 or 2, wherein
the migratory cell-free cell layer contains migration target cells, and
the migration target cells are cells that determine the migration direction of the migratory cells.

7. The method for evaluating the migration ability of migratory cells according to claim 1 or 2, wherein
the migratory cell-free cell layer contains no migration target cell, and
the migration target cells are cells that determine the migration direction of the migratory cells.

8. The method for evaluating the migration ability of migratory cells according to claim 1 or 2, wherein
the culture step is performed using, as the cell structure, each of a first cell structure in which the migratory cell-free cell layer contains migration target cells, and a second cell structure in which the migratory cell-free cell layer contains no migration target cell,
in the evaluation step, the migration ability of the migratory cells is evaluated based on the mobility of the migratory cells in the first cell structure and the mobility of the migratory cells in the second cell structure, and
the migration target cells are cells that determine the migration direction of the migratory cells.

9. The method for evaluating the migration ability of migratory cells according to claim 6, wherein
the method also evaluates the cytotoxicity of the migratory cells against the migration target cells.

10. The method for evaluating the migration ability of migratory cells according to claim 6, wherein
the migration target cells are cancer cells.

11. The method for evaluating the migration ability of migratory cells according to claim 1 or 2, wherein
the height Hs of the cell structure is 50 µm or more.

12. A cell structure comprising a stromal cell layer containing migratory cells and stromal cells, and a migratory cell-free cell layer containing no migratory cells on a top surface of the stromal cell layer,
the migratory cell-free cell layer containing migration target cells, and
the migration target cells being cells that determine the migration direction of the migratory cells.

13. A cell structure comprising a stromal cell layer containing migratory cells and stromal cells, and a migratory cell-free cell layer containing no migratory cells on a top surface of the stromal cell layer,
the migratory cell-free cell layer containing no migration target cells, and
the migration target cells being cells that determine the migration direction of the migratory cells.
